# EUROPÄISCHE PATENTSCHRIFT

(11) **EP 3 151 000 B1**
(45) Veröffentlichungstag und Bekanntmachung des Hinweises auf die Patenterteilung: **14.07.2021**
(21) Anmeldenummer: 16002306.5
(22) Anmeldetag: 01.09.2014
(51) Int. Cl.: G01N 27/404, G01N 33/00

(54) **FLÜSSIGER ELEKTROLYT FÜR EINEN ELEKTROCHEMISCHEN GASSENSOR**
LIQUID ELECTROLYTE FOR AN ELECTROCHEMICAL GAS SENSOR
ÉLECTROLYTE LIQUIDE POUR UN CAPTEUR DE GAZ ÉLECTROCHIMIQUE

(30) Priorität: 09.09.2013 DE 102013014995
(43) Veröffentlichungstag der Anmeldung: 05.04.2017
(62) Teilanmeldung aus: 14758300.9
(73) Patentinhaber: Dräger Safety AG & Co. KGaA, 23560 Lübeck (DE)
(72) Erfinder: NAUBER, Andreas, 23617 Stockelsdorf (DE); SICK, Michael, 23669 Timmendorfer Strand (DE); STEINER, Gregor, 79822 Titisee-Neustadt (DE); MATTERN-FRÜHWALD, Marie-Isabell, 22941 Bargteheide (DE); CHRZAN, Rigobert, 23843 Bad Oldesloe (DE); SOMMER, Sabrina, 23558 Lübeck (DE); METT, Frank, 23556 Lübeck (DE); HENGSTENBERG, Andreas, 23858 Reinfeld (DE)
(74) Vertreter: Kettenbeil, Roxane Henriette

(56) Entgegenhaltungen:
- EP-A1- 2 224 018
- WO-A1-2013/045561
- GB-A- 2 225 859
- XIAOBO JI ET AL: "Determination of ammonia based on the electrochemical oxidation of N,N'-diphenylenediamine in propylene carbonate", ANALYTICAL SCIENCES, Bd. 23, November 2007 (2007-11), Seiten 1317-1320, XP055154055,
- LOPEZ DE MISHIMA B A ET AL: "Ammonia sensor based on propylene carbonate", SENSORS AND ACTUATORS B: CHEMICAL, Bd. 131, Nr. 1, 14. April 2008 (2008-04-14), Seiten 236-240, XP022602889, ISSN: 0925-4005, DOI: 10.1016/J.SNB.2007.11.012
- GIOVANELLI D ET AL: "Determination of ammonia based on the electro-oxidation of hydroquinone in dimethylformamide or in the room temperature ionic liquid, 1-ethyl-3-methylimidazolium bis(trifluoromethylsulfonyl)imide", TALANTA, Bd. 62, Nr. 5, April 2004 (2004-04), Seiten 904-911, XP055153888, ISSN: 0039-9140, DOI: 10.1016/j.talanta.2003.10.015
- Anon: "Good's buffers", , 1 January 2017 (2017-01-01), XP055423303, Retrieved from the Internet: URL:https://en.wikipedia.org/api/rest_v1/p age/pdf/Good's_buffers [retrieved on 2017-11-09]

## Beschreibung

Die Erfindung betrifft einen flüssigen Elektrolyt für einen elektrochemischen Gassensor, insbesondere für einen elektrochemischen Gassensor zum Nachweis von NH₃ oder NH₃-haltigen Gasgemischen.

Elektrochemische Gassensoren, mit denen über einen begrenzten Zeitraum die Konzentration von gasförmigem Ammoniak (NH₃) detektierbar ist, sind allgemein bekannt. Solche Sensoren kommen üblicherweise in den verschiedensten technischen Bereichen zum Einsatz, von der chemischen Industrie über die Überwachung von Kühlanlagen bis hin zu landwirtschaftlichen Betrieben. Sie dienen insbesondere dazu kritische Konzentrationen des entzündlichen, bei Einatmung giftigen und ätzenden Ammoniak-Gases rechtzeitig zu erkennen und vor einer entsprechenden Gefahr zu warnen.

Eines der wesentlichen Bestandteile eines solchen elektrochemischen Sensors ist der in dem Sensor verwendete Elektrolyt. Der Elektrolyt steht dabei in leitendem Kontakt mit wenigstens einer Anode und einer Kathode. Tritt das nachzuweisende Gas in den elektrochemischen Sensor ein, so findet typischerweise zwischen dem Gas, dem Elektrolyten und dem Elektrodenmaterial eine Reaktion statt, die zu einem messbaren Stromfluss zwischen der Anode und der Kathode des Sensors führt.

So beschreibt EP 0 395 927 B1 eine elektrochemische Messzelle zur Bestimmung von Ammoniak oder Hydrazin in einer gasförmigen oder flüssigen Messprobe mit mindestens einer Messelektrode und einer Gegenelektrode, die in einer mit einem lösliche Elektrolyten gefüllten Elektrolytkammer aufgenommen sind, welche zur Messprobe hin durch eine permeable Membran abgeschlossen ist.

Auch EP 0 556 558 B1 sieht eine elektrochemische Messzelle zur Bestimmung von Ammoniak, Aminen, Hydrazin und Hyrdazinderivaten vor. Hier wird vorgeschlagen, als Leitelektrolyten in der Elektrolytlösung ein hygroskopisches Alkali- oder Erdalkalisalz zu verwenden. Dies soll ein Austrocknen des Elektrolyten verhindern und auf diese Weise eine möglichst langfristige Verwendbarkeit des Sensors ermöglichen.

Die GB 2225859A offenbart einen Elektrolyten für die Bestimmung von Ammoniak. Der Elektrolyt enthält tris-(Hydroxymethyl)-Aminomethan Hydrochloride und ein hygroscopisches Additiv aus gewählt aus Tetramethyl-Ammonium Chloride oder Lithium Chloride.

Der Nachweise von Ammoniak (NH₃) bei derart gestalteten elektrochemischen Sensoren erfolgt mit Hilfe einer elektrochemischen Reaktion zwischen dem in den Sensor einströmenden Ammoniakgas, den Elektroden und dem Elektrolyten des Sensors. Im Zuge dieser Reaktion wird eintretendes Ammoniakgas an der Messelektrode oxidiert. Die dabei entstehenden AmmoniumIonen werden anschließend an der Gegenelektrode wieder deprotoniert. In diesem Zusammenhang kann es sich aber zum Beispiel als problematisch erweisen, dass als Nebenprodukt dieser Reaktion weitere Stickstoffverbindungen gebildet werden können, die zu einer Blockierung (Vergiftung) der Elektrodenoberflächen führen kann.

Ausgehend davon ist es Aufgabe der vorliegenden Erfindung, diese und andere Nachteile des Standes der Technik zu überwinden.

Als Lösung sieht die Erfindung einen flüssigen Elektrolyten entsprechend Anspruch 1 vor. Ausgestaltungen sind Gegenständer abhängigen Ansprüche.

Bei einem flüssigen Elektrolyt für einen elektrochemischen Gassensor, insbesondere für einen elektrochemischen Gassensor, der zum Nachweis von NH₃ oder NH₃-haltigen Gasgemischen geeignet ist, sieht die Erfindung vor, dass der Elektrolyt wenigstens ein Lösungsmittel, ein Leitsalz und/oder einen organischen Mediator enthält, wobei das Leitsalz eine ionische Flüssigkeit, ein anorganisches Salz, ein organisches Salz oder ein Gemisch daraus ist.

Insbesondere für elektrochemische Gassensoren, bei welchen Elektroden aus Edelmetall oder Kohlenstoffnanoröhren verwendet werden, kann ein solcher Elektrolyt mit großem Vorteil eingesetzt werden, um die Dauerbegasungsfestigkeit eines solchen Sensors zu verbessern. Insbesondere das Risiko einer wie oben beschriebenen Vergiftung kann auf diese Weise deutlich minimiert werden.

Erfindungsgemäss enthält der Elektrolyt einen Puffer entsprechend der

Formel I R¹-(CR²R³)ₙ-SO₃H

mit n= 1, 2, 3, 4 oder 5, bevorzugt n = 2 oder n = 3 ist, wobei alle R² und R³ unabhängig voneinander ausgewählt sind aus H, NH und OH, und wobei R¹ ausgewählt ist aus der Gruppe enthaltend Piperazinyl, substituiertes Piperazinyl, N-morpholino, Cylcoalkyl, tris-(Hydroxyalkyl)alkyl. Beispielsweise können R² und R³ unabhängig voneinander ausgewählt sein aus H, NH und OH, wobei n = 2 ist und R¹ ausgewählt ist aus der Gruppe enthaltend Piperazinyl, substituiertes Piperazinyl, N-morpholino, Cylcoalkyl, tris-(Hydroxyalkyl)alkyl. Vorstellbar ist zum Beispiel auch, dass R² und R³ unabhängig voneinander ausgewählt sind aus H, NH und OH, wobei n = 2 ist und R¹ ausgewählt ist aus der Gruppe enthaltend N-morpholino und tris-(Hydroxyalkyl)alkyl). Beispielsweise ist es dabei ganz besonders von Vorteil, wenn n = 2 oder n = 3 ist, wobei alle R² und R³ unabhängig voneinander ausgewählt sind aus H, NH und OH, und wobei R¹ ausgewählt ist aus [4-(2-Hydroxyethyl)-1]-piperazinyl, (N-morpholino), N-Cyclohexyl, tris-(Hydroxymethyl)methyl. Ganz besonders bevorzugt ist der Puffer 3-(N-morpholino)-propansulfonsäure oder 3-(N-morpholino)ethansulfonsäure. So ist es zum Beispiel denkbar, dass der Elektrolyt ein Gemisch aus einem Lösungsmittel, einem Leitsalz und/oder einem organischen Mediator ist, wobei das Leitsalz eine ionische Flüssigkeit, ein anorganisches Salz, ein organisches Salz oder ein Gemisch daraus ist, und wobei der Elektrolyt außerdem einen Puffer enthält, insbesondere einen Puffer, der ausgewählt ist aus 3-(N-morpholino)-propansulfonsäure oder 3-(N-morpholino)-ethansulfonsäüre.

Um zu verhindern, dass der Elektrolyt nach einer gewissen Zeit austrocknet - z.B. wenn der Sensor im Dauerbetrieb verwendet werden soll - ist es außerdem vorteilhaft, wenn der Elektrolyt als weitere Komponente eine Komponente zur Erniedrigung des Dampfdruckes enthält. Dabei kann die weitere Komponente bevorzugt ein Alkylenglykol oder Polyalkylenglykol sein, besonderes bevorzugt Propylenglykol, Ethylenglykol oder ein Gemisch aus Propylenglykol und Ethylenglykol ist. So ist es zum Beispiel denkbar, dass der Elektrolyt ein Gemisch aus einem Lösungsmittel, einem Leitsalz und/oder einem organischen Mediator ist, wobei das Leitsalz eine ionische Flüssigkeit, ein anorganisches Salz, ein organisches Salz oder ein Gemisch daraus ist und wobei der Elektrolyt außerdem wenigstens ein Alkylenglykol enthält, insbesondere ein Alkylenglykol, das ausgewählt ist aus Propylenglykol, Ethylenglykol oder einem Gemisch aus Proylenglykol und Ethylenglykol.

Weiterhin ist es günstig, wenn das Lösungsmittel ausgewählt ist aus der Gruppe enthaltend Wasser und Alkylencarbonat oder Gemische daraus, bevorzugt ausgewählt aus der Gruppe enthaltend Wasser, Propylencarbonat, Ethylencarbonat oder Gemische daraus. Denkbar ist beispielsweise, dass der Elektrolyt ein Gemisch aus einem Lösungsmittel, einem Leitsalz und/oder einem organischen Mediator ist, wobei das Leitsalz eine ionische Flüssigkeit, ein anorganisches Salz, ein organisches Salz oder ein Gemisch daraus ist und wobei das Lösungsmittel Wasser ist. Alternativ ist auch vorstellbar, dass der Elektrolyt ein Gemisch aus einem Lösungsmittel, einem Leitsalz und/oder einen organischen Mediator ist, wobei das Leitsalz eine ionische Flüssigkeit, ein anorganisches Salz, ein organisches Salz oder ein Gemisch daraus ist und wobei das Lösungsmittel Alkylencarbonat, insbesondere Propylencarbonat, Ethylencarbonat oder ein Gemisch aus Propylencarbonat und Ethylencarbonat ist. Dabei ist es insbesondere auch vorstellbar, dass der Elektrolyt ein Gemisch aus einem Lösungsmittel, einem Leitsalz und/oder einem organischen Mediator ist, wobei das Leitsalz eine ionische Flüssigkeit, ein anorganisches Salz, ein organisches Salz oder ein Gemisch daraus ist, wobei der Elektrolyt außerdem einen Puffer enthält, insbesondere einen Puffer, der ausgewählt ist aus 3-(N-morpholino)-propansülfonsäure oder 3-(N-morpholino)-ethansulfonsäure und wobei das Lösungsmittel Alkylencarbonat, insbesondere Propylencarbonat, Ethylencarbonat oder ein Gemisch aus Propylencarbonat und Ethylencarbonat ist. Außerdem ist es denkbar, dass der Elektrolyt ein Gemisch aus einem Lösungsmittel, einem Leitsalz und/oder einem organischen Mediator ist, wobei das Leitsalz eine ionische Flüssigkeit, ein anorganisches Salz, ein organisches Salz oder ein Gemisch daraus ist, wobei der Elektrolyt außerdem wenigstens ein Alkylenglykol enthält, insbesondere ein Alkylenglykol, das ausgewählt ist aus Propylenglykol, Ethylenglykol oder einem Gemisch aus Proylenglykol und Ethylenglykol, und wobei Lösungsmittel Alkylencarbonat, insbesondere Propylencarbonat, Ethylencarbonat oder ein Gemisch aus Propylencarbonat und Ethylencarbonat ist.

Bevorzugt ist das Anion des Leitsalzes ausgewählt aus der Gruppe enthaltend Halogenide, Carbonat, Sulfonat, Phosphat und/oder Phosphonat, bevorzugt ein Anion ausgewählt aus der Gruppe enthaltend Alkyl-sulfonat, Alkenyl-Sulfonat, Aryl-Sulfonat, Alkyl-phosphat, Alkenyl-Phosphat, Aryl-Phosphat, substituiertes Alkyl-sulfonat, substituiertes Alkenyl-sulfonat, substituiertes Aryl-sulfonat, substituiertes Alkyl-phosphat, substituiertes Alkenyl-phosphat, substituiertes Aryl-phosphat, halogeniertes Phosphat, halogeniertes Sulfonat halogeniertes Alkyl-sulfonat, halogeniertes Alkenyl-sulfonat, halogeniertes Aryl-sulfonat, halogeniertes Alkyl-phosphat, halogeniertes Alkenyl-phosphat, halogeniertes Aryl-phosphat, besonders bevorzugt ein Anion ausgewählt aus der Gruppe enthaltend Fluorophosphat, Alkylfluorophosphat, Aryl-sulfonat, ganz besonders bevorzugt aus der Gruppe enthaltend Perfluoralkylfluorophosphat, Toluolsulfonat.

Dabei ist es vorteilhaft, wenn das Leitsalz als Kationen Metallionen, Oniumionen oder ein Gemisch aus Metallionen und Oniumionen enthält. Zum Beispiel können die Metallionen ausgewählt sein aus Alkalimetallionen oder Erdalkalimetallionen, bevorzugt aus Li, K und/oder Na. Günstig ist es, wenn die Oniumionen ausgewählt sind aus Ammonium-, Phosphonium, Guanidiniumkationen und heterocyclischen Kationen, bevorzugt ausgewählt aus AlkylAmmonium- und heterocyclischen Kationen, besonders bevorzugt ausgewählt aus AlkylAmmonium, Imidazolium und/oder substituierten Imidazoliumionen, wobei die substituierten Imidazolium-Ionen bevorzugt eine Struktur entsprechend 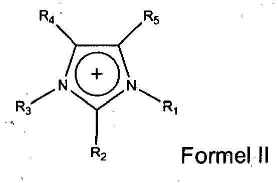 aufweisen, wobei R1, R2, R3, R4 und R5 unabhängig voneinander ausgewählt sein können aus -H, geradkettiges oder verzweigtes Alkyl mit 1 bis 20 C-Atomen, geradkettiges oder verzweigtes Alkenyl mit 2 bis 20 C-Atomen und einer oder mehreren Doppelbindungen, geradkettiges oder verzweigtes Aklinyl mit 2 bis 20 C-Atomen und einer oder mehreren Dreifachbindungen, gesättigtes, teilweise oder vollständig ungesättigtes Cycloalkyl mit 3-7 C-Atomen, das mit Alkylgruppen mit 1 bis 6 C-Atomen substituiert sein kann, gesättigtes teilweise oder vollständig ungesättigtes Heteroaryl, Heteroaryl-C1-C6-alkyl oder Aryl-C1-C6-alkyl, wobei besonders bevorzugt R2, R4 und R5 H sind und R1 und R3 jeweils unabhängig voneinander ein geradkettiges oder verzweigtes Alkyl mit 1 bis 20 C-Atomen.

Beispielsweise ist insbesondere vorstellbar, dass als Leitsalz Tetrabutlyammoniumtoluolsulfonat oder 1-Hexyl-3-methyl-imidazolium-tris(pentafluoroethyl)-trifluorophosphat verwendet wird. Alternativ ist auch vorstellbar, dass das Leitsalz zum Beispiel LiCI, KCl- oder eine Mischung aus LiCI und KCl ist. So ist es insbesondere vorteilhaft, wenn der Elektrolyt ein Gemisch aus einem Lösungsmittel, einem Leitsalz und/oder einem organischen Mediator ist, wobei das Leitsalz ausgewählt ist aus LiCI, KCl, Alkylammonium-toluolsulfonat und ionischen Flüssigkeiten, mit einem Perfluoralkylfluorophosphat-Anion.

Weiterhin ist es günstig, wenn der organische Mediator eine Polyhydroxyverbindung ist, welche bei Oxidation ein chinoides System oder ein Naphthalin-System bildet. Beispielsweise kann der organische Mediator ausgewählt sein aus der Gruppe enthaltend ortho-Dihydroxybenzol, para-Dihydroxybenzol, substituierte ortho-Dihydroxybenzole und substituierte para-Dihydroxybenzole, Dihydroxynaphtalin, substituiertes Dihydroxynaphtalin, Anthrahydrochinon, substituiertes Anthrahydrochinon, bevorzugt 1,2-Dihydroxybenzol, 1,4 Dihydroxybenzol, Naphtohydrochinon, substituiertes 1,2- oder 1,4.Dihydroxybenzol, substituiertes Hydrochinon, substituiertes Naphtohydrochinon, besonders bevorzugt substituiertes Anthrahydrochinon, substituiertes Hydrochinon, substituiertes 1,2-Dihydroxybenzol. Dabei ist es besonders günstig, wenn die Substituenten des substituierten Anthrachinons, substituierten 1,2-Dihydroxybenzol und/oder substituierten 1,4-Hydrochinon ausgewählt sind aus der Gruppe enthaltend Sulfonyl, tert-Butyl, Hydroxy, Alkyl, Aryl, bevorzugt Sulfonsäure und/oder tert-Butyl.

In jedem Fall ist es besonders günstig, wenn der Elektrolyt als Lösungsmittel ein Gemisch aus Propylencarbonat und/oder Ethylencarbonat aufweist, als Leitsalz LiCI, KCl, Tetrabutylammoniumtoluolsulfonat und/oder 1-Hexyl-3-methyl-imidazolium-tris(pentafluoroethyl)-trifluorophosphat oder ein Gemisch aus zwei oder mehr dieser Komponenten aufweist und als organischen Mediator tert-butyl-Hydrochinon und/oder ein substituiertes Anthrachinon, bevorzugt Anthrachinon-2-Sulfonat aufweist.

Die Konzentration des organischen Mediators kann dabei zwischen 10-6 mol/ und 10-2 ml/l liegen. So kann der organische Mediator in einer Konzentration von 10⁻² mol/l oder weniger, bevorzugt von 10⁻³ mol/l oder weniger, besonders bevorzugt von 5^{∗}10⁻⁴ mol/l oder weniger, ganz besonders bevorzugt von 2^{∗}10⁻⁴ mol/l oder weniger, ganz besonders bevorzugt von 10⁻⁴ mol/l oder weniger in dem Elektrolyt enthalten sein. Denkbar ist auch, dass der organische Mediator in einer Konzentration von 10⁻⁶ mol/l oder mehr, bevorzugt von 10⁻⁵ mol/l oder mehr, besonders bevorzugt von 5^{∗}10⁻⁵ mol/l oder mehr, ganz besonders bevorzugt von 8^{∗}10⁻⁵ mol/l oder mehr, ganz besonders bevorzugt von 10⁻⁴ mol/l oder mehr in dem Elektrolyt enthalten ist. Insbesondere ist es auch denkbar, dass der organische Mediator in einer Konzentration von 10⁻⁵ mol/l bis 10⁻³ mol/l, bevorzugt 5^{∗}10⁻⁵ mol/l bis 5^{∗}10⁻⁴ mol/l, besonders bevorzugt 8^{∗}10⁻⁵ mol/l bis 2^{∗}10⁻⁴ mol/l, ganz besonders bevorzugt 10⁻⁴ mol/l vorhanden ist.

Ein erfindungsgemäßer Elektrolyt ist besonders bevorzugt erhältlich mit Hilfe eines Verfahrens, welches die folgenden Schritte umfasst:
a. Vorlegen des Lösungsmittels in einem Reaktionsgefäß
b. Zugabe des Puffers
c. Zugabe des organischen Mediators
d. Erhitzen des Gemisches unter Rühren für ca. 15 Minuten auf 150 °C
e. Rühren für etwa eine Stunde ohne weitere Hitzezufuhr bis alle Feststoffe gelöst sind
f. Abkühlen auf Raumtemperatur
g. Zugabe des Leitsalzes

Weitere Details und Einzelheiten ergeben sich aus den nachfolgend beschriebenen Figuren und Ausführungsbeispielen. Dabei zeigt
- Fig. 1: einen schematischen Aufbau eines elektrochemischen Gassensors mit welchem der erfindungsgemäße Elektrolyt zum Nachweis von Ammoniak verwendbar ist.
- Fig. 2: einen schematischen Ablauf einer Nachweisreaktion für NH₃ in einem elektrochemischen Gassensor, welcher einen erfindungsgemäßen Elektrolyten enthält.

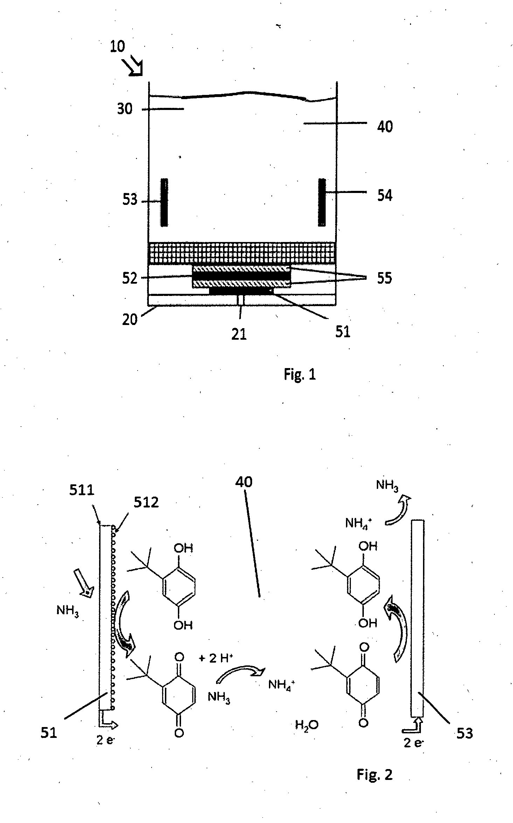

Man erkennt in Fig. 1, einen elektrochemischen Gassensor 10, welcher ein Gehäuse 20 mit einem Elektrolytreservoir 30 aufweist. In dem Gehäuse 20 ist ein Gaseinlass 21 und ein Gasauslass 22 ausgebildet. Innerhalb des Gehäuses 20 ist eine Arbeitselektrode 51 derart angeordnet, das sie in Kontakt mit Gas steht, das durch den Gaseinlass 21 in das Gehäuse 20 einströmt. Die Arbeitselektrode 51 ist mit Hilfe einer Glasfasermembran 55 von einer Abfangelektrode 52 getrennt. Die Abfangelektrode 52 ist ihrerseits mit einer Glasfasermembran 55 von dem Elektrolytreservoir 30 getrennt. Innerhalb des Elektrolytreservoirs 30 sind weiterhin eine Gegenelektrode 53 und eine Referenzelektrode 54 angeordnet.

In dem Elektrolytreservoir 30 ist der erfindungsgemäße Elektrolyt 40 vorhanden. Dabei sind die Glasfasermembranen 55 mit dem Elektrolyten durchtränkbar. Auf diese Weise kann der Elektrolyt 40 sowohl zur Arbeitselektrode 51 als auch zur Abfangelektrode 52 gelangen, so dass dort jeweils eine chemische Reaktion entsprechend dem in Fig. 2 dargestellten Schema zwischen einströmendem NH₃, dem Material der Arbeits- bzw. Abfangelektrode 51, 52 und dem Elektrolyten 40 stattfinden kann:

Dabei reagiert in den Gassensor 10 einströmendes NH3 an der Oberfläche der Arbeitselektrode 51 mit dem Elektrolyten. Bevorzugt besteht die Arbeitselektrode 51 dabei z.B. aus einer PTFE-Membran mit einer Kohlenstoff-Nanoröhren-Beschichtung. Die Gegenelektode 53 besteht bevorzugt aus einem Edelmetall. Der Elektrolyt 40 ist in diesem Beispiel eine Zusammensetzung aus Propylencarbonat und/oder Ethylencarbonat als Lösungsmittel, 1-Hexyl-3-methyl-imidazolium-tris(pentafluoroethyl)-trifluorophosphat als Leitsalz und tert-butyl-1, 2-Dihydroxybenzol als organischem Mediator. Der Elektrolyt enthält weiterhin bevorzugt einen Puffer, nämlich 3-(N-morpholino)propansulfonsäure. Wie in Fig. 2 erkennbar, wird das tert-Butyl-1,2-Dihydroxybenzol an der Arbeitselektode 51 zu tert-butyl-Ghinon oxidiert. Die dabei freigesetzten Protonen reagieren mit dem in den Gassensor 10 einströmenden NH3 zu Ammoniumionen. Die Ammoniumionen gelangen zur Gegenelektrode 53, wo die Rückreaktion des zuvor gebildeten tert-butyl-Chinon zu 1,2-Dihydroxybenzol stattfindet. Dabei wird aus den Ammoniumionen wiederum NH₃ freigesetzt, das durch den Gasauslass 22 entweichen kann. Im Zuge dieses Reaktionsablaufes stabilisiert der eingesetzte Puffer den pH-Wert des Elektrolyten, der zwischen der Arbeits- und der Gegenelektrode 51, 53 im Elektrolytreservoir 30 vorliegt.

Ausführungsbeispiel für die Herstellung eines erfindungsgemäßen Elektrolyten:
In einem Reaktionsgefäß wird als Lösungsmittel Polycarbonat vorgelegte. Zu dem Polycarbonat wird 0,4 Gew.% Puffer, bevorzugt 3-(N-morpholinop)propansulfonsäure zugegeben. Im nächsten Schritt werden 6,9 Gew.% des organischen Mediators, bevorzugt tert-butyl-1,2-Dihydroxybenzol zugegeben. Die Mischung wird unter Rühren innerhalb von 15 Minuten erhitzt, wobei eine maximale Temperatur von 150°C nicht überschritten wird. Im Anschluss wurde die Mischung für eine Stunde ohne weitere Hitzezufuhr weiter gerührt bis alle Feststoffe gelöst waren. Die erhaltene Lösung hat eine klare, leicht gelbliche Farbe.

Die so erhaltene Lösung wird stehen gelassen, bis sie auf Raumtemperatur abgekühlt ist. Danach werden 2,7 Gew.% des Leitsalzes, bevorzugt Hmim-FAP (3-Hexyl-3-methyl-imidazolium-tris(pentafluoroethyl)-trifluorphosphat), zugegeben und das Gemisch wird kurz, für etwa 1 Minute, umgerührt.

**Bezugszeichenliste**

| | | | |
|---|---|---|---|
| 10 | Gassensor | 40 | Elektrolyt |
| | | | |
| 20 | Gehäuse | 51 | Arbeitselektrode |
| 21 | Gaseinlass | 52 | Abfangelektrode |
| 22 | Gasauslass | 53 | Gegenelektrode |
| | | 54 | Referenzelektrode |
| 30 | Elektrolytreservoir | 55 | Glasfasermembran |

## Patentansprüche

1. Flüssiger Elektrolyt für einen elektrochemischen Gassensor, insbesondere für einen elektrochemischen Gassensor, der zum Nachweis von NH3 oder NH₃-haltigen Gasgemischen geeignet ist, wobei der Elektrolyt wenigstens ein Lösungsmittel, ein Leitsalz und einen organischen Mediator enthält, wobei das Leitsalz eine ionische Flüssigkeit, ein anorganisches Salz, ein organisches Salz oder ein Gemisch daraus ist, **dadurch gekennzeichnet, dass** der Elektrolyt einen Puffer enthält, wobei der Puffer eine Verbindung entsprechend
Formel I R¹-(CR²R³)ₙ-SO₃H
mit n= 1, 2, 3, 4 oder 5, bevorzugt n = 2 oder n = 3 ist, wobei alle R² und R³ unabhängig voneinander ausgewählt sind aus H, NH und OH, und wobei R¹ ausgewählt ist aus der Gruppe enthaltend Piperazinyl, substituiertes Piperazinyl, N-morpholino, Cylcoalkyl, tris-(Hydroxyalkyl)alkyl.

2. Elektrolyt entsprechend Anspruch 1, **dadurch gekennzeichnet, dass** n = 2 oder n = 3 ist, wobei alle R² und R³ unabhängig voneinander ausgewählt sind aus H, NH und OH, und wobei R1 ausgewählt ist aus [4-(2-Hydroxyethyl)-1]-piperazinyl, (N-morpholino), N-Cyclohexyl, tris-(Hydroxymethyl)methyl, wobei der Puffer bevorzugt 3-(N-morpholino)propansulfonsäure oder 3-(N-morpholino)ethansulfonsäure ist.

3. Elektrolyt entsprechend einem der Ansprüche 1 oder 2, **dadurch gekennzeichnet, dass** der Elektrolyt als weitere Komponente eine Komponente zur Erniedrigung des Dampfdruckes enthält, wobei die weitere Komponente bevorzugt ein Alkylenglykol oder Polyalkylenglykol ist, besonderes bevorzugt Propylenglykol, Ethylenglykol oder ein Gemisch aus Propylenglykol und Ethylenglykol ist.

4. Elektrolyt entsprechend wenigstens einem der Ansprüche 1 bis 3, **dadurch gekennzeichnet, dass** das Lösungsmittel ausgewählt ist aus der Gruppe enthaltend Wasser und Alkylencarbonat oder Gemische daraus, bevorzugt ausgewählt aus der Gruppe enthaltend Wasser, Propylencarbonat, Ethylencarbonat oder Gemische daraus.

5. Elektrolyt entsprechend wenigstens einem der Ansprüche 1 oder 4, **dadurch gekennzeichnet, dass** das Anion des Leitsalzes ausgewählt ist aus der Gruppe enthaltend Halogenide, Carbonat, Sulfonat, Phosphat und/oder Phosphonat.

6. Elektrolyt entsprechend wenigstens einem der Ansprüche 1 bis 5, **dadurch gekennzeichnet, dass** das Leitsalz als Kationen Metallionen, Oniumionen oder ein Gemisch aus Metallionen und Oniumionen enthält.

7. Elektrolyt entsprechend Anspruch 6, **dadurch gekennzeichnet, dass** die Metallionen ausgewählt sind aus Alkalimetallionen oder Erdalkalimetallionen, bevorzugt aus Li, K und/oder Na.

8. Elektrolyt entsprechend einem der Ansprüche 6 oder 7, **dadurch gekennzeichnet, dass** die Oniumionen ausgewählt sind aus Ammonium-, Phosphonium, Guanidiniumkationen und heterocyclischen Kationen.

9. Elektrolyt entsprechend einem der Ansprüche 1 bis 8, **dadurch gekennzeichnet, dass** der organische Mediator eine Polyhydroxyverbindung ist, welche bei Oxidation ein chinoides System oder ein Naphthalin-System bildet.

10. Elektrolyt entsprechend Anspruch 9, **dadurch gekennzeichnet, dass** der organische Mediator ausgewählt ist aus der Gruppe enthaltend ortho-Dihydroxybenzol, para-Dihydroxybenzol, substituierte ortho-Dihydroxybenzole und substituierte para-Dihydroxybenzole, Dihydroxynaphtalin, substituiertes Dihydroxynaphtalin, Anthrahydrochinon, substituiertes Anthrahydrochinon.

11. Elektrolyt entsprechend Anspruch 10, **dadurch gekennzeichnet, dass** die Substituenten des substituierten Anthrachinons, substituierten 1,2-Dihydroxybenzol und/oder substituierten 1,4-Hydrochinon ausgewählt sind aus der Gruppe enthaltend Sulfonyl, tert-Butyl, Hydroxy, Alkyl, Aryl, bevorzugt Sulfonsäure und/oder tert-Butyl.

12. Elektrolyt entsprechend einem der Ansprüche 1 bis 11, **dadurch gekennzeichnet, dass** der Elektrolyt als Lösungsmittel ein Gemisch aus Propylencarbonat und/oder Ethylencarbonat aufweist, als Leitsalz LiCI, KCl, Tetrabutylammoniumtoluolsulfonat und/oder 1-Hexyl-3-methyl-imidazolium-tris(pentafluoroethyl)-trifluorophosphat oder ein Gemisch aus zwei oder mehr dieser Komponenten aufweist und als organischen Mediator tert-butyl-Hydrochinon und/oder ein substituiertes Anthrachinon, bevorzugt Anthrachinon-2-Sulfonat aufweist.

13. Elektrolyt entsprechend einem der Ansprüche 1 bis 12, **dadurch gekennzeichnet, dass** der organische Mediator in einer Konzentration von 10⁻² mol/l oder weniger, bevorzugt von 10⁻³ mol/l oder weniger, besonders bevorzugt von 5^{∗}10⁻⁴ mol/l oder weniger, ganz besonders bevorzugt von 2^{∗}10⁻⁴ mol/l oder weniger, ganz besonders bevorzugt von 10⁻⁴ mol/l oder weniger in dem Elektrolyt enthalten ist.

14. Elektrolyt entsprechend einem der Ansprüche 1 bis 13, **dadurch gekennzeichnet, dass** der organische Mediator in einer Konzentration von 10⁻⁶ mol/l oder mehr, bevorzugt von 10⁻⁵ mol/l oder mehr, besonders bevorzugt von 5^{∗}10⁻⁵ mol/l oder mehr, ganz besonders bevorzugt von 8^{∗}10⁻⁵ mol/l oder mehr, ganz besonders bevorzugt von 10⁻⁴ mol/l oder mehr in dem Elektrolyt enthalten ist.

15. Verfahren zur Herstellung eines Elektrolyten entsprechend einem der vorhergehenden Ansprüche, umfassend die Schritte
a. Vorlegen des Lösungsmittels in einem Reaktionsgefäß
b. Zugabe des Puffers
c. Zugabe des organischen Mediators
d. Erhitzen des Gemisches unter Rühren für ca. 15 Minuten auf 150 °C
e. Rühren für etwa eine Stunde ohne weitere Hitzezufuhr bis alle Feststoffe gelöst sind
f. Abkühlen auf Raumtemperatur
g. Zugabe des Leitsalzes

## Claims

1. Liquid electrolyte for an electrochemical gas sensor, especially for an electrochemical gas sensor suitable for detection of NH₃ or NH₃-containing gas mixtures, wherein the electrolyte comprises at least one solvent, a conductive salt and an organic mediator, wherein the conductive salt is an ionic liquid, an inorganic salt, an organic salt or a mixture thereof, **characterized in that** the electrolyte comprises a buffer, wherein the buffer is a compound of
Formula I R¹- (CR²R³)ₙ-SO₃H
with n = 1, 2, 3, 4 or 5, preferably n = 2 or n = 3, where all R² and R³ are independently selected from H, NH and OH, and where R¹ is selected from the group comprising piperazinyl, substituted piperazinyl, N-morpholino, cycloalkyl, tris(hydroxyalkyl)alkyl.

2. Electrolyte according to Claim 1, **characterized in that** n = 2 or n = 3, where all R² and R³ are independently selected from H, NH and OH, and where R¹ is selected from [4-(2-hydroxyethyl)-1]-piperazinyl, (N-morpholino), N-cyclohexyl, tris-(hydroxymethyl)methyl, where the buffer is preferably 3-(N-morpholino)propanesulfonic acid or 3-(N-morpholino)ethanesulfonic acid.

3. Electrolyte according to either of Claims 1 and 2, **characterized in that** the electrolyte comprises, as a further component, a component for lowering the vapour pressure, where the further component is preferably an alkylene glycol or polyalkylene glycol, more preferably propylene glycol, ethylene glycol or a mixture of propylene glycol and ethylene glycol.

4. Electrolyte according to at least one of Claims 1 to 3, **characterized in that** the solvent is selected from the group comprising water and alkylene carbonate or mixtures thereof, preferably selected from the group comprising water, propylene carbonate, ethylene carbonate or mixtures thereof.

5. Electrolyte according to at least one of Claims 1 and 4, **characterized in that** the anion of the conductive salt is selected from the group comprising halides, carbonate, sulfate, phosphate and/or phosphonate.

6. Electrolyte according to at least one of Claims 1 to 5, **characterized in that** the conductive salt contains, as cations, metal ions, onium ions or a mixture of metal ions and onium ions.

7. Electrolyte according to Claim 6, **characterized in that** the metal ions are selected from alkali metal ions and alkaline earth metal ions, preferably from Li, K and/or Na.

8. Electrolyte according to either of Claims 6 and 7, **characterized in that** the onium irons are selected from ammonium cations, phosphonium cations, guanidinium cations and heterocyclic cations.

9. Electrolyte according to any of Claims 1 to 8, **characterized in that** the organic mediator is a polyhydroxyl compound that forms a quinoid system or a naphthalene system on oxidation.

10. Electrolyte according to Claim 9, **characterized in that** the organic mediator is selected from the group comprising ortho-dihydroxybenzene, para-dihydroxybenzene, substituted ortho-dihydroxybenzenes and substituted para-dihydroxybenzenes, dihydroxynaphthalene, substituted dihydroxynaphthalene, anthrahydroquinone, substituted anthrahydroquinone.

11. Electrolyte according to Claim 10, **characterized in that** the substituents of the substituted anthraquinone, substituted 1,2-dihydroxybenzene and/or substituted 1,4-hydroquinone are selected from the group comprising sulfonyl, tert-butyl, hydroxyl, alkyl, aryl, preferably sulfonic acid and/or tert-butyl.

12. Electrolyte according to any of Claims 1 to 11, **characterized in that** the electrolyte includes a mixture of propylene carbonate and/or ethylene carbonate as solvent, LiCl, KCl, tetrabutylammonium toluenesulfonate and/or 1-hexyl-3-methylimidazolium tris(pentafluoroethyl)trifluorophosphate or a mixture of two or more of these components as conductive salt, and tert-butylhydroquinone and/or a substituted anthraquinone, preferably anthraquinone-2-sulfonate, as organic mediator.

13. Electrolyte according to any of Claims 1 to 12, **characterized in that** the organic mediator is present in the electrolyte in a concentration of 10⁻² mol/l or less, preferably of 10⁻³ mol/l or less, more preferably of 5^{∗}10⁻⁴ mol/l or less, even more preferably of 2^{∗}10⁻⁴ mol/l or less, even more preferably of 10⁻⁴ mol/l or less.

14. Electrolyte according to any of Claims 1 to 13, **characterized in that** the organic mediator is present in the electrolyte in a concentration of 10⁻⁶ mol/l or more, preferably of 10⁻⁵ mol/l or more, more preferably of 5^{∗}10⁻⁵ mol/l or more, even more preferably of 8^{∗}10⁻⁵ mol/l or more, even more preferably of 10⁻⁴ mol/l or more.

15. Process for preparing an electrolyte according to any of the preceding claims, comprising the steps of
a. initially charging the solvent in a reaction vessel
b. adding the buffer
c. adding the organic mediator
d. heating the mixture to 150°C while stirring for about 15 minutes
e. stirring for about one hour without further supply of heat until all solids have dissolved
f. cooling to room temperature
g. adding the conductive salt.

## Revendications

1. Électrolyte liquide pour un capteur électrochimique de gaz, en particulier pour un capteur électrochimique de gaz qui est approprié à la détection de NH₃ ou de mélanges de gaz contenant du NH₃, l'électrolyte contenant au moins un solvant, un sel conducteur et un médiateur organique, le sel conducteur étant un liquide ionique, un sel inorganique, un sel organique ou un mélange de ceux-ci, **caractérisé en ce que** l'électrolyte contient un tampon, le tampon étant un composé correspondant à la
formule I R¹-(CR²R³)ₙ-SO₃H
où n = 1, 2, 3, 4 ou 5, de préférence n = 2 ou n = 3, tous les radicaux R² et R³ étant choisis indépendamment les uns des autres parmi H, NH et OH, et R¹ étant choisi dans le groupe contenant les groupes pipérazinyle, pipérazinyle substitué, N-morpholino, cycloalkyle, tris(hydroxyalkyl)alkyle.

2. Électrolyte selon la revendication 1, **caractérisé en ce que** n = 2 ou n = 3, tous les radicaux R² et R³ étant choisis indépendamment les uns des autres parmi H, NH et OH, et R¹ étant choisi parmi les groupes [4-(2-hydroxyéthyl)-1]-pipérazinyle, (N-morpholino), N-cyclohexyle, tris-(hydroxyméthyl)méthyle, le tampon étant de préférence l'acide 3-(N-morpholino)propane-sulfonique ou l'acide 3-(N-morpholino)éthanesulfonique.

3. Électrolyte selon l'une quelconque des revendications 1 et 2, **caractérisé en ce que** l'électrolyte contient comme autre composant un composant destiné à l'abaissement de la tension de vapeur, l'autre composant étant de préférence un alkylèneglycol ou polyalkylèneglycol, de façon particulièrement préférée le propylèneglycol, l'éthylèneglycol ou un mélange de propylèneglycol et d'éthylèneglycol.

4. Électrolyte selon au moins l'une quelconque des revendications 1 à 3, **caractérisé en ce que** le solvant est choisi dans le groupe contenant l'eau et un carbonate d'alkylène ou des mélanges de ceux-ci, de préférence choisi dans le groupe contenant l'eau, le carbonate de propylène, le carbonate d'éthylène ou des mélanges de ceux-ci.

5. Électrolyte selon au moins l'une quelconque des revendications 1 et 4, **caractérisé en ce que** l'anion du sel conducteur est choisi dans le groupe contenant des halogénures, un carbonate, sulfonate, phosphate et/ou phosphonate.

6. Électrolyte selon au moins l'une quelconque des revendications 1 à 5, **caractérisé en ce que** le sel conducteur contient comme cations des ions métalliques, des ions onium ou un mélange d'ions métalliques et d'ions onium.

7. Électrolyte selon la revendication 6, **caractérisé en ce que** les ions métalliques sont choisis parmi des ions de métaux alcalins ou des ions de métaux alcalino-terreux, de préférence parmi Li, K et/ou Na.

8. Électrolyte selon l'une quelconque des revendications 6 et 7, **caractérisé en ce que** les ions ionium sont choisis parmi les cations ammonium, phosphonium, guanidinium et des cations hétérocycliques.

9. Électrolyte selon l'une quelconque des revendications 1 à 8, **caractérisé en ce que** le médiateur organique est un composé polyhydroxy qui forme à l'oxydation un système quinoïde ou un système naphtalénique.

10. Électrolyte selon la revendication 9, **caractérisé en ce que** le médiateur organique est choisi dans le groupe contenant l'ortho-dihydroxybenzène, le para-dihydroxybenzène, les ortho-dihydroxybenzènes substitués et para-dihydroxybenzènes substitués, le dihydroxynaphtalène, un dihydroxynaphtalène substitué, l'anthrahydroquinone, une anthrahydroquinone substituée.

11. Électrolyte selon la revendication 10, **caractérisé en ce que** les substituants de l'anthraquinone substituée, du 1,2-dihydroxybenzène substitué et/ou de la 1,4-hydroquinone substituée sont choisis dans le groupe contenant des substituants sulfonyle, tert-butyle, hydroxy, alkyle, aryle, de préférence sulfonyle et/ou tert-butyle.

12. Électrolyte selon l'une quelconque des revendications 1 à 11, **caractérisé en ce que** l'électrolyte comporte comme solvant un mélange de carbonate de propylène et/ou carbonate d'éthylène, comme sel conducteur LiCl, KCl, le toluènesulfonate de tétrabutylammonium et/ou le tris-(pentafluoroéthyl)-trifluorophosphate de 1-hexyl-3-méthyl-imidazolium ou un mélange de deux ou plus de deux de ces composants et comme médiateur organique la tert-butyl-hydroquinone et/ou une anthraquinone substituée, de préférence l'anthraquinone-2-sulfonate.

13. Électrolyte selon l'une quelconque des revendications 1 à 12, **caractérisé en ce que** le médiateur organique est contenu dans l'électrolyte à une concentration de 10⁻² mole/l ou moins, de préférence de 10⁻³ mole/l ou moins, de façon particulièrement préférée de 5^{∗}10⁻⁴ mole/l ou moins, de façon tout particulièrement préférée de 2^{∗}10⁻⁴ mole/l ou moins, de façon tout particulièrement préférée de 10⁻⁴ mole/l ou moins.

14. Électrolyte selon l'une quelconque des revendications 1 à 13, **caractérisé en ce que** le médiateur organique est contenu dans l'électrolyte à une concentration de 10⁻⁶ mole/l ou plus, de préférence de 10⁻⁵ mole/l ou plus, de façon particulièrement préférée de 5^{∗}10⁻⁵ mole/l ou plus, de façon tout particulièrement préférée de 8^{∗}10⁻⁵ mole/l ou plus, de façon tout particulièrement préférée de 10⁻⁴ mole/l ou plus.

15. Procédé pour la préparation d'un électrolyte selon l'une quelconque des revendications précédentes, comprenant les étapes
a. disposition au préalable du solvant dans un récipient de réaction
b. addition du tampon
c. addition du médiateur organique
d. chauffage du mélange sous agitation pendant environ 15 minutes à 150 °C
e. agitation pendant environ une heure sans autre apport de chaleur, jusqu'à ce que tous les solides soient dissous
f. refroidissement jusqu'à la température ambiante
g. addition du sel conducteur.
